# FASCICULE DE BREVET EUROPEEN

(11) **EP 1 212 017 B1**
(45) Date de publication et mention de la délivrance du brevet: **02.08.2006**
(21) Numéro de dépôt: 00956744.7
(22) Date de dépôt: 13.09.2000
(51) Int. Cl.: A61F 5/00

(54) **ANNEAU DE GASTROPLASTIE**
RING ZUR VERFORMUNG DES MAGENS
GASTROPLASTY RING

(30) Priorité: 14.09.1999 FR 9911618
(43) Date de publication de la demande: 12.06.2002
(73) Titulaire: SURGICAL-IOC, 42000 Saint-Etienne (FR)
(72) Inventeur: LATOUR, Marie-Jeanne, B-8094 Bertrange (LU)
(74) Mandataire: Dupuis, François
(86) Numéro de dépôt international: PCT/IB2000/001293
(87) Numéro de publication internationale: WO 2001/019297

(56) Documents cités:
- DE-A- 19 751 733
- US-A- 4 696 288

## Description

L'objet de l'invention se rattache au secteur technique des dispositifs chirurgicaux introduits dans le corps humain pour le traitement de l'obésité morbide.

Depuis de nombreuses années, on a mis au point des techniques chirurgicales pour traiter l'obésité morbide.

Parmi celles-ci, on peut citer l'introduction dans l'estomac d'un ballon intragastrique entraînant une sensation rapide de satiété lors de la prise alimentaire, le court circuitage de la quasi totalité de l'intestin par une anastomose entre le jéjunum et l'iléon, la dérivation bilio-pancréatique, techniques ont été rapidement abandonnées car peu efficaces dans le temps et sources de nombreuses complications.

On s'est alors orienté vers la chirurgie de restriction gastrique, c'est-à-dire la création d'une petite poche dans la partie proximale de l'estomac communiquant avec le reste de l'estomac par un orifice calibré. Selon cette technique, lorsque le patient ingurgite des aliments, la petite poche se remplit très vite induisant un sentiment de satiété et obligeant le patient à manger lentement afin de laisser le temps à cette poche de se vider.

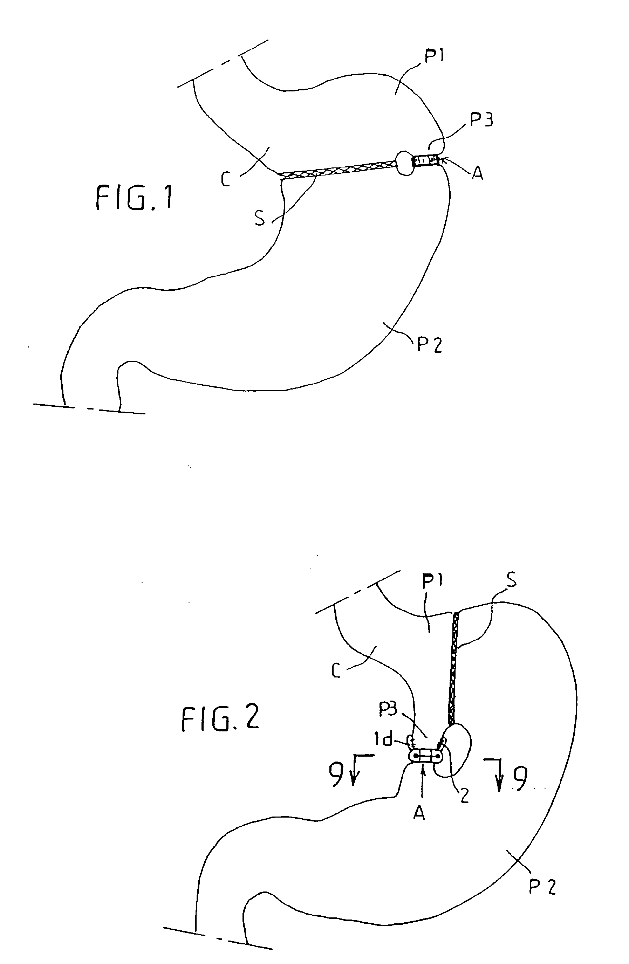

Pour cela diverses techniques opératoires ont été mises en oeuvre, et notamment la gastroplastie horizontale, et la gastroplastie verticale de l'estomac, c'est-à-dire, comme le montrent les figures 1 et 2., la création à partir du cardia (C) (orifice entre l'estomac et l'oesophage) d'une poche (P1) de petites dimensions par séparation puis suture (S) et calibrage de l'orifice entre la petite poche (P1) et le reste de l'estomac formant la poche (P2) par mise en place d'un anneau (A) en élastomère biocompatible agrafé sur la paroi gastrique ou simplement placé sur la paroi externe de l'estomac et dont on peut régler le diamètre intérieur par gonflage.

On connaît ainsi de telles anneaux de gastroplastie décrit dans le brevet US 4696288 et le brevet DE 19751733.

Cette technique peu invasive (par coelioscopie ou laparatomie) est satisfaisante dans l'ensemble. Cependant, il peut arriver que cet anneau se vrille sur lui-même ou bien se déplace par basculement entraînant au besoin une nouvelle intervention, ce qui, on le conçoit n'est pas très rationnel et pénible pour le patient. Des recherches ont été entreprises pour améliorer cette technique.

L'anneau de gastroplastie selon l'invention remédie à ces inconvénients en ce qu'il est parfaitement positionné autour de l'orifice séparant les deux poches de l'estomac, sans possibilité de migration, et qu'il est facile à mettre en place.

Pour cela et selon une première caractéristique, l'anneau comprend des agencements permettant la liaison ferme par sutures de l'anneau avec ladite paroi gastrique, après déformation de l'anneau. Le réglage du diamètre de passage entre les deux poches ainsi formées est obtenu par des fils de suture introduits dans l'anneau et ligaturés à chaque extrémité dudit anneau, après introduction par l'oesophage d'un organe de calibrage. L'orifice longitudinal du tube réalisé dans l'épaisseur de l'anneau est en communication avec un orifice central établi à partir de la génératrice opposée au méplat, afin d'introduire les fils de suture non résorbables ligaturés ou autrement reliés à leurs extrémités arrières au niveau dudit orifice et débouchant aux extrémités de l'anneau afin d'être noués en vue de la fixation dudit anneau en position.

Ces caractéristiques et d'autres encore ressortiront de la description qui suit.

Pour fixer l'objet de l'invention, sans toutefois le limiter, dans les dessins annexés :
- Les figures 1 et 2 illustrent respectivement une pose d'anneau par gastroplastie horizontale et par gastroplastie verticale, selon l'art antérieur.

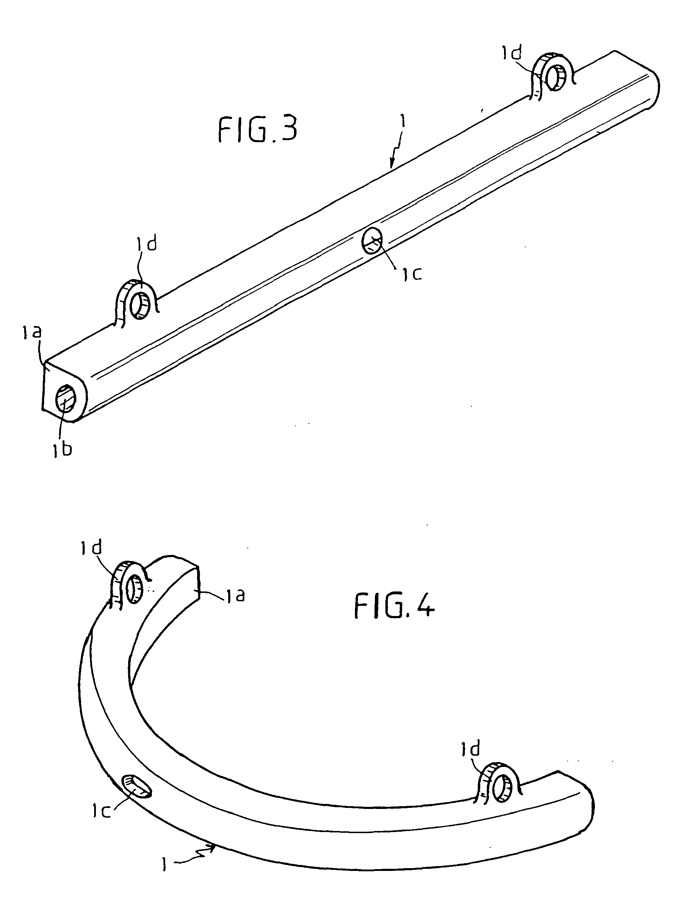

- La figure 3 est une vue en perspective montrant un anneau selon l'invention dans sa position repos.
- La figure 4 est une vue en perspective montrant un anneau selon l'invention en position recourbée.

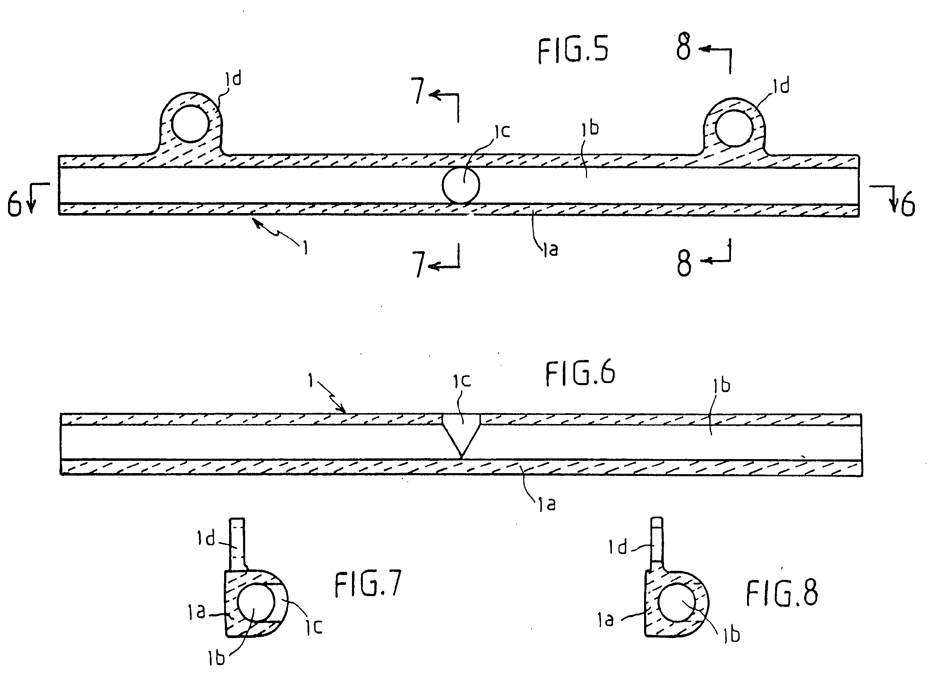

- La figure 5 est une vue en coupe longitudinale de l'anneau selon l'invention.
- La figure 6 est une vue de dessus et en coupe considérée selon la ligne 6-6 de la figure 5.
- Les figures 7 et 8 sont des vues en coupe transversale considérées respectivement selon les lignes 7-7 et 8-8 de la figure 5.
- La figure 9 est une vue en coupe considérée selon la ligne 9-9 de la figure 2 montrant la mise en place de l'anneau selon l'invention au niveau de la partie d'estomac à restreindre.
- La figure 10 est une vue en coupe partielle correspondant à la figure 9, après calibrage et ligature de l'anneau.

Afin de rendre plus concret l'objet de l'invention, on le décrit maintenant sous une forme non limitative de réalisation illustrée aux figures des dessins.

Comme indiqué dans le préambule, on réalise par coelioscopie ou laparotomie, une partition verticale par viscerosynthèse au niveau de la petite courbure gastrique de l'estomac, soit directement par application d'une pince linéaire, soit après avoir réalisé une anastomose transgastrique circulaire, puis on met en place, après l'avoir purgé dans une solution stérile, l'anneau selon l'invention.

Cet anneau (1) est réalisé en matériau souple, déformable et bio compatible tel qu'un élastomère de silicone sous la forme d'un tube de section générale circulaire, mais présentant sur toute sa longueur (environ 50mm) un méplat (1a) qui constitue la face d'appui et de contact avec la paroi gastrique lorsque l'anneau est mis en place autour de ladite paroi.

Dans sa partie médiane, l'orifice longitudinal (1b) formant canal établi dans l'épaisseur de l'anneau est en communication avec un orifice central (1c) établi à partir de la génératrice opposée au méplat. Enfin, près de ses extrémités, le tube présente parallèlement au méplat au moins deux oreillettes ou boutonnières (Id), en léger retrait du méplat.

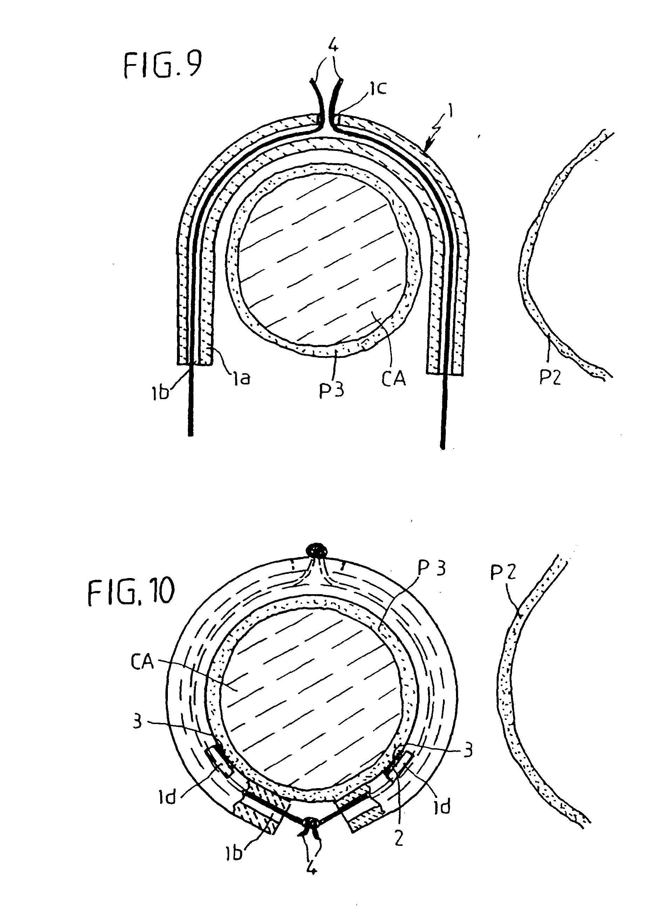

Comme on le voit bien aux figures 9 et 10 des dessins, après avoir mis en place, par déformation élastique et courbure, l'anneau autour de la paroi gastrique (P3) séparant les deux poches (P1, P2) précitées constituées après anastomose, et après avoir introduit par l'oesophage un organe de calibrage (CA) du type tube de Faucher, on relie l'anneau à la paroi gastrique (P3) par des points de suture (2) à l'aide de fils non résorbables (3) attachés aux boutonnières (1d). Puis des fils (4) également non résorbables, sont engagés par l'orifice central (1c) et débouchent aux deux extrémités de l'anneau, par le biais de l'orifice (1b). Ces fils sont alors noués à leur extrémité arrière au niveau dudit orifice central, puis l'anneau est refermé sur lui-même et solidement assujetti à la paroi par tension et nouage ou sertissage des extrémités débordant de l'anneau sous le contrôle de l'organe de calibrage, afin de réaliser le rétrécissement souhaité.

Les avantages ressortent bien de la description, on souligne encore la parfaite tenue de l'anneau par suture des oreillettes à la paroi gastrique évitant toute bascule, et par le méplat évitant l'effet de twist ou vrillage. La possibilité de reprendre ultérieurement l'intervention sans grosses complications pour modifier le rétrécissement ou l'annuler du fait de la technique de pose non invasive. '

La configuration intérieure de l'anneau peut être établie avec toutes formes appropriées. Le nombre d'oreillettes peut être établi d'une manière variable. La fabrication de l'anneau est aisée en ce sens qu'il est réalisé en bandes rectilignes secables selon un pas préétabli. Cette déformation élastique lui permet de s'adapter à toutes morphologies de l'estomac ou des individus. Sa position est stable par suite de sa partie méplate qui ne glisse pas sur la paroi de l'estomac.

## Revendications

1. Anneau de gastroplastie apte à être disposé autour d'une paroi gastrique (P3) formée par séparation verticale de l'estomac du côté de sa courbure interne en partie proximale, puis suture afin de constituer une petite poche (P1) et une poche (P2) avec le reste de l'estomac, l'anneau (1),étant réalisé en un matériau élastiquement déformable, sous la forme d'un tube l'anneau comprend des agencements (1d) permettant la liaison ferme par sutures (3) de l'anneau avec ladite paroi gastrique, après déformation de l'anneau,
et en ce que le réglage du diamètre de passage entre les deux poches ainsi formées peut être obtenu par des fils de suture (4) introduits dans l'anneau et ligaturés à chaque extrémité dudit anneau, après introduction par l'oesophage d'un organe de calibrage (CA), **caractérisé en ce que** ledit tube présente sur toute sa longueur un méplat (1a) destiné à prendre appui contre la paroi gastrique (P3) à étreindre
et **en ce que** l'orifice longitudinal (1b) du tube réalisé dans l'épaisseur de l'anneau est en communication avec un orifice central (1c) établi à partir de la génératrice opposée au méplat (1a), afin de permettre l'introduction des fils de suture non résorbables (4) ligaturés ou autrement reliés à leurs extrémités arrière au niveau dudit orifice et débouchant aux extrémités de l'anneau afin d'être noués en vue de la fixation dudit anneau en position.

2. Anneau selon la revendication 1, **caractérisé en ce que** les agencements de liaison ferme de l'anneau avec la paroi gastrique rétreinte (P3) sont constitués par des oreillettes ou boutonnières (1d) établies parallèlement au méplat (1a) et légèrement en retrait dudit méplat, lesdites oreillettes ou boutonnières étant solidarisées à ladite paroi par des fils de suture non résorbables (3).

3. Anneau selon la revendication 1, **caractérisé en ce qu'**il est réalisé en matériau biocompatible du type élastomère de silicone.

4. Anneau selon la revendication 1 **caractérisé en ce qu'**il est fabriqué sous forme de bandes rectilignes et secables.

## Claims

1. Gastric band capable of being fitted around a stomach wall (P3) constituted by a vertical separation of the stomach on the side of the internal curve in the proximal section, then sutured in order to create a small cavity (P1) and a cavity (P2) with the rest of the stomach, the band (1) being made of an elastically distortable material as a tube, the band comprising fittings (1d) that enable the band to be firmly secured to said stomach wall via sutures (3) after distortion of the band, and the diameter of the passage between the two cavities thus defined being capable of being set using suture threads (4) that are introduced into the band and tied at each end of said band after a calibrating apparatus (CA) has been introduced via the oesophagus, **characterised in that** said tube has over its entire length a flat edge (1a) intended to bear against the stomach wall (P3) to be constricted, and **in that** the longitudinal orifice (1b) of the tube formed in the body of the band communicates with a central orifice (1c) formed from the generating line opposite the flat edge (1a), in order to make it possible to introduce the non-resorbable suture threads (4) the rear ends of which are tied or otherwise connected in the zone around said orifice and leave by the ends of the band in order to be tied to enable said band to be fastened into position.

2. Band of claim 1, **characterised in that** the fittings that secure the band firmly to the restricted stomach wall (P3) consist of lugs or eyelets (1d) provided parallel to the flat edge (1a) and slightly set back from said flat edge, said lugs or eyelets being connected to said wall by non-resorbable suture threads (3).

3. Band of claim 1, **characterised in that** it is made of a silicon elastomer-type biocompatible material.

4. Band of claim 1, **characterised in that** it is manufactured in straight strips that can be cut.

## Patentansprüche

1. Ring zur Verformung des Magens, der um eine Magenwand (P3) herum angeordnet werden kann, die durch vertikale Trennung des Magens auf der Seite seiner Innenkrümmung am proximalen Abschnitt gebildet wird, und anschließende Naht, so dass eine kleine Tasche (P1) und eine Tasche (P2) mit dem Rest des Magens gebildet wird, wobei der Ring (1) aus einem elastisch verformbaren Material in Form eines Rohres hergestellt wird, das über seine gesamte Länge eine Abflachung (1a) besitzt, die sich gegen die abzuschnürende Magenwand (P3) abstützt, **dadurch gekennzeichnet, dass** der Ring Anordnungen (1d) besitzt, die die dauerhafte Verbindung des Rings mit der Magenwand durch Nähte (3) nach der Verformung des Rings erlauben, und dass man die Einstellung des Durchgangsquerschnitts zwischen den beiden auf diese Art und Weise geformten Taschen durch Nahtfäden (4) erhalten kann, die in den Ring eingeführt und nach Einführung eines Kalibrierorgans (CA) über die Speiseröhre an jedem Ende des Rings gebunden werden, **dadurch gekennzeichnet, dass** das Rohr über seine gesamte Länge eine Abflachung (1a) besitzt, die sich gegen die abzudrehende Magenwand (P3) abstützt, und dass die Längsöffnung (1b) des Rohres, welche die Stärke des Ringes besitzt, mit einer mittleren Öffnung (1c) in Verbindung steht, die ausgehend von der, der Abflachung (1a) gegenüberliegenden, Mantellinie (1a) gebildet wird, um so die Einführung nicht absorbierbarer (4) Nahtfäden zu ermöglichen, die an ihren hinteren Enden im Bereich der Öffnung gebunden oder anderweitig verbunden sind und in die Enden des Rings münden, um im Hinblick auf die feste Positionierung des Rings verknotet zu werden.

2. Ring nach Anspruch 1, **dadurch gekennzeichnet, dass** die festen Verbindungsanordnungen des Rings mit der zugezogenen Magenwand (P3) aus Ösen und länglichen Aussparungen (1d) bestehen, die parallel zu der Abflachung (1a) und leicht zurückgesetzt von der Abflachung gebildet werden, wobei die Ösen und länglichen Aussparungen durch nicht absorbierbare Nahtfäden (3) mit der Wand verbunden sind.

3. Ring nach Anspruch 1, **dadurch gekennzeichnet, dass** er aus einem biokompatiblen Material wie einem SilikonElastomer hergestellt wird.

4. Ring nach Anspruch 1, **dadurch gekennzeichnet, dass** er in Form von geraden und teilbaren Bändern hergestellt wird.
